# EUROPÄISCHE PATENTANMELDUNG

(11) **EP 0 752 423 A1**
(43) Veröffentlichungstag der Anmeldung: **08.01.1997**
(21) Anmeldenummer: 96110360.3
(22) Anmeldetag: 27.06.1996
(51) Int. Cl.: C07D 307/92, C11B 9/00, B01J 21/04

(54) **Verfahren zur stereoselektiven Herstellung von (-)3a,6,6,9a-Tetramethyl-perhydronaphtho[2,1-b]furan**

(30) Priorität: 06.07.1995 DE 19524584
(71) Anmelder: BASF AKTIENGESELLSCHAFT, 67056 Ludwigshafen (DE)
(72) Erfinder: Grimmer, Johannes, 67061 Ludwigshafen (DE); Martin, Christoph, Dr., 68165 Mannheim (DE)

(57) **Zusammenfassung**

Verfahren zur stereoselektiven Herstellung von (-)3a,6,6,9a-Tetramethyl-perhydronaphtho[2,1-b]furan der Formel Ia 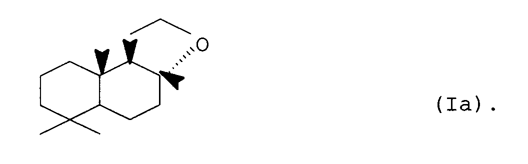 durch Dehydratisierung und Cyclisierung von Decahydro-2-hydroxy-2,5,5,8a-tetramethyl-1-naphthalinethanol mittels festen sauren Katalysatoren, das dadurch gekennzeichnet ist, daß man das Decahydro-2-hydroxy-2,5,5,8a-tetramethyl-1-naphthalinethanol in geschmolzenem Zustand in Gegenwart von 10 % bis 100 Gew-%, bezogen auf das Diol, an einem kommerziell für die (präparative) Säulenchromatographie angebotenen aktiven sauren Aluminiumoxid auf Temperaturen zwischen 80 und 200°C erhitzt.

## Beschreibung

Die Erfindung betrifft ein Verfahren zur stereoselektiven Herstellung von (-) 3a,6,6,9a-Tetramethyl-perhydronaphtho[2,1-b]furan der Formel Ia

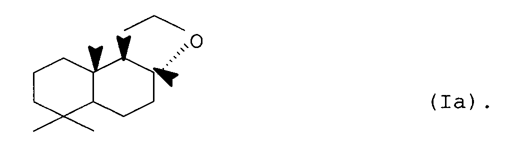

Diese Verbindung stellt einen seit Jahrzehnten in der Parfümerie verwendeten Ambra-Riechstoff (Ambrox® der Fa. Firmenich; Amboxan® der Fa. Henkel und Sylvamber® der Fa. BASF) dar.

Dieser Ambra-Riechstoff wurde inzwischen neben seinem Vorkommen in der Ambra-Tinktur des Pottwales ebenfalls in Muskateller-Salbei-Öl (Salvia Sclarea L.), im Labdanum-Öl (Cistus labdaniferus L.) sowie in Cypressen-Öl (Cupressus semperoirens L.) nachgewiesen (vgl. G. Ohloff in Fragrance Chemistry, Academic Press, 1982, s. 543). Er ruft den in Parfüm-Kompositionen begehrten "Ambra-Effekt" hervor, der auch in großer Verdünnung wirksam ist und sorgt darüber hinaus für eine ausgezeichnete Fixierung auch zarter, blumiger Düfte. Er wird hauptsächlich in teuren Parfümölen eingesetzt.

Die vier Diastereomeren dieses Ambra-Riechstoffs 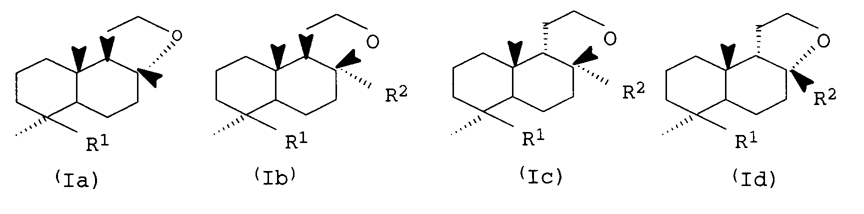 Ia-Id unterscheiden sich deutlich voneinander. So ist z.B. der Geruch des sogenannten "Isoambrox" (Ib) über hundertmal schwächer als der der Verbindung der Formel Ia (vgl. G. Ohloff et al. i. Helv. Chimica Acta, Vol. 68 (1985), S. 2022 bis 2029) und der Geruch des sogenannten "Norisoambrox" (Formel Ib mit R¹ = H und R² = CH₃) über 500 mal schwächer als der der Verbindung der Formel Ia.

Der Ambra-Riechstoff der Formel Ia bzw. die Diasteromeren-Gemische der Formeln Ia bis Id lassen sich sowohl partialsynthetisch aus Ambrein oder aus Diterpenen vom Labdan-Typ (z.B. Sclareol, Manool) als auch durch Totalsynthese herstellen (vgl. Übersicht von E.-J. Brunke, Dragoco-Report, 11/12 (1979) S. 276 ff).

Im technischen Maßstab wird der Ambra-Riechstoff hauptsächlich durch oxidativen Abbau (Chromsäure, Permanganat, Ozon) von Sclareol, einem Inhaltsstoff des Muskateller Salbei Concretes, hergestellt. Das als Abbau-Produkt erhaltene Sclareolid wird nach einer Lithiumalanat- oder Natriumboranat-Reduktion in ein 1,4-Diol, ("Ambroxdiol") übergeführt, das anschließend cyclisiert wird (vgl. G. Ohloff, Fortschr. Chem. Forsch. 12/2, (1969) 185 ff).

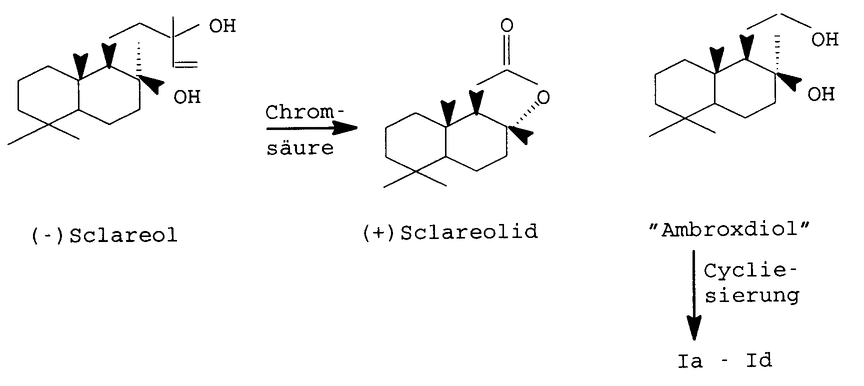

Aufgrund der Tatsache, daß unter den vier Diastereomeren die Verbindung der Formel Ia die größte olfaktorische Bedeutung besitzt, wurden in der Vergangenheit zahlreiche Verfahren entwickelt, die versuchten den letzten Schritt, die Cyclisierung, stereoselektiv durchzuführen.

So beschrieben V.E. Sibiertseva et al. in Maslo-Zhir, Prom.-st., 1979 (12), S. 25 bis 26, die Cyclisierung des "Ambroxdiols" in Gegenwart von p-Toluolsulfonsäure. Nachteilig an dem Verfahren ist die im sauren Medium nicht zu vermeidende Dehydratisierung der tertiären Hydroxygruppe, die zu Selektivitätsverlusten führt. Außerdem sind die angegebenen Ausbeuten von 55 bis 60 % bei einem technischen Verfahren unbefriedigend, insbesondere unter Berücksichtigung des teuren Edukts.

Die Verwendung von p-Toluolsulfonsäure wird auch in den Verfahren der russischen Patente SU 345 183 (von 1968), SU 910 561 (von 1980) sowie SU 529 166 (von 1975) beansprucht.

In dem Verfahren gemäß einem älteren Patent der Fa. Firmenich (DP 860 214 von 1949) wurden als Katalysatoren Naphthalinsulfonsäure (78 % Ausbeute) sowie Aluminiumoxid (50 % Ausbeute) beschrieben. Auch hierbei wurden die oben aufgeführten Nachteile, wie schlechte Selektivität, sowie unbefriedigende Ausbeuten beobachtet.

Gemäß dem Verfahren des spanischen Patents 432 815 (von 1976) wurde die Cyclisierung des "Ambroxdiols" mit Schwefelsäure durchgeführt; die hierbei erzielten Ausbeuten lagen bei nur 29 %.

R.C. Cambie et al. beschrieben in Aust. J. Chem. 24 (1971), S. 583 bis 591 sowie S. 2365 bis 2377, die Cyclisierung des "Ambroxdiols" mittels p-Toluolsulfonylchlorid in Pyridin mit 80 % Ausbeute. Hauptnachteile sind in diesem Falle der Einsatz des unangenehm riechenden Pyridins im Hinblick auf die Qualitätseinstellung des erhaltenen Riechstoffes sowie die relativ langen Reaktionszeiten der Umsetzung. Darüber hinaus ist bei der wäßrigen Aufarbeitung eine Pyridin-Rückführung durch dessen Wasserlöslichkeit unumgänglich, was zu einer Kostensteigerung führt.

Cambie verwendete bei einer zweiten Cyclisierungsmethode Schwefelsäure als Katalysator. Nachteilig sind hierbei die lange Reaktionszeit (>3 Tage) sowie die schlechten Ausbeuten (43 % der Theorie).

Auch bei dem vom Consertium Elektrochemie in der DOS 3 240 054 (von 1982) beschriebenen Cyclisierungsverfahren mit POCl₃ arbeitete man in wasserfreiem Pyridin, und man mußte daher die oben beschriebenen Nachteile in Kauf nehmen. Die Ausbeuten betrugen nur 65 % d. Theorie.

P.F. Vead and N.D. Unger beschrieben in "Synthesis", 1983, S. 816 bis 818 eine Cyclisierungsmethode, bei der als Reagens Trimethylchlorsilan in Dimethylsulfoxid (DMSO) eingesetzt wurde; die hierbei angegebenen Ausbeuten betragen 85 %. Wie aus dem hierin aufgeführten experimentellen Beispiel hervorgeht, stellt diese Methode nur ein Laborverfahren der (mg-Ansätze). Für die Technik von Nachteil ist der Einsatz des DMSO, das nach der wäßrigen Aufarbeitung aufgrund seiner Wasserlöslichkeit (Abwasserprobleme) aufwendig recyclisiert werden muß. Ein entscheidender Nachteil ist darüber hinaus die Notwendigkeit einer Reinigung des nach der Reaktion erhaltenen rohen Ambra-Riechstoffs auf eine sowohl chemisch als auch olfaktorisch akzeptierbare Qualität. Die chemische Reinigung erfolgte durch kostspielige Säulenchromatographie, wobei eine vollständige DMSO-Entfernung meist schwierig ist. Ein weiteres Problem ist das technisch häufig schwierige Handling von Trimethylchlorsilan, welches sehr korrosiv wirkt und außerdem toxikologisch bedenklich ist.

Die gleiche Cyclisierungsmethode wird auch im russischen Patent SU 988 817 (von 1980) beschrieben.

Weiterhin ist aus EP-A-204 009 ein Verfahren zur Herstellung von Ambrox® bekannt, bei dem Sclareol auf biotechnischem Wege in das sogenannte "Ambroxdiol" überführt und dieses zum (-)3a,6,6,9a-Tetramethyl-perhydronaphtho[2,1-b]furan der Formel Ia cyclisiert wird. Die Cyclisierung wird mit einem Arylsulfonylchlorid in Gegenwart von sauren Verbindungen, wie HCl und sauren Ionenaustauschern oder in Gegenwart von Basen, wie Pyridin und NaOH vorgenommen. Bei der hier beschriebenen Cyclisierung in saurem Milieu werden schlechte Ausbeuten erzielt. Die Nachteile einer Cyclisierung in Gegenwart von organischen Basen, wie Pyridin im technischen Maßstab, wurden oben ausführlich erläutert.

Relativ gut gelingt die Cyclisierung mit Sulfonylchloriden in Gegenwart von Alkalihydroxiden. Nachteilig hierbei sind jedoch die relativ langen Reaktionszeiten; die noch ungenügenden Reinheiten der erhaltenen Verbindung der Formel Ia sowie die teilweise sehr hohe Reaktionstemperatur, die zu Selektivitätsverlusten führen kann.

Aus Chem. Abstr. 105, 134193 K (1986), ist weiterhin bekannt, daß bei der Verwendung von Weißerde, Tonerde oder Kieselerde, die mit 1 bis 20 Gew.-% Schwefelsäure, Phosphorsäure oder Polyphosphorsäure beladen sind, als Katalysatoren die theoretischen Ausbeuten an der Verbindung der Formel Ia auf 85 - 90,5 % gesteigert werden können. In 2 Beispielen wird die Reinheit der Verbindung der Formel Ia mit 97 % und 98 % angegeben.

Aus DE 39 12 318 ist weiterhin ein Verfahren zur Cyclisierung von "Ambroxdiol" in Gegenwart von säurebeladenen Aluminiumoxiden als Katalysatoren bekannt, welches durch folgende Merkmale charakterisiert ist:
1) Verwendung von 60 bis 80 Gew.-% Al₂O₃ bezogen auf "Ambroxdiol"
2) Salzsäurebeladung zwischen 0,4 und 0,6 Gew.-%
3) Schüttdichte 0,9 g/cm³
4) Kornbereich 0,05 bis 0,2 mm

Nachteilig hierbei sind jedoch, daß die Cyclisierung nicht in einem für die olfaktorischen Ansprüche ausreichendem Maße stereoselektiv ist und daß man ganz spezielle, mit Salzsäure vorbehandelte Aluminiumoxidkatalysatoren verwenden muß.

Nachteilig an allen vorgenannten Verfahren ist die Unverzichtbarkeit auf Lösungsmittel, welche bei der olfaktorischen Bewertung einen negativen Einfluß haben können, weil sie vom Wertprodukt sehr leicht adsorbiert werden und nur durch weitere kostpielige Maßnahmen, wie Wasserdampfdestillation oder verlängerte Trocknungszeiten entfernbar sind.

Es bestand somit die Aufgabe, ein hochselektives Cyclisierungsverfahren aufzufinden, das technisch auf einfache Weise durchführbar ist und das ohne die Nachteile der Verfahren des Standes der Technik zu einem olfaktorisch geeigneten Produkt führt.

Gegenstand der Erfindung ist ein Verfahren zur stereoselektiven Herstellung von (-) 3a, 6,6,9a-Tetramethyl-perhydronaphtho[2,1-b]-furan der Formel Ia 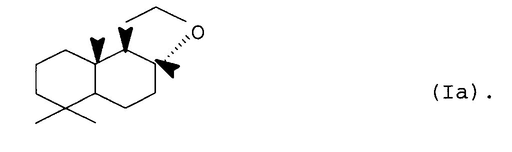 durch Dehydratisierung und Cyclisierung von Decahydro-2-hydroxy-2,5,5,8-tetramethyl-1-naphthalinethanol mittels festen sauren Katalysatoren, das dadurch gekennzeichnet ist, daß man das Decahydro-2-hydroxy-2,5,5,8-tetramethyl-1-naphthalinethanol im geschmolzenen zustand für 1 bis 6 Stunden in Gegenwart von 10 bis 100 Gew.-%, bezogen auf das Diol, an einem kommerziell für die (präparative) Säulenchromatographie angebotenen aktiven sauren Aluminiumoxid auf Temperaturen von 80 bis 180°C, vorzugsweise 120 bis 140°C erhitzt.

Das als Ausgangsverbindung verwendete Diol Decahydro-2-hydroxy-2,5,5,8-tetramethyl-1-naphthalinethanol ist eine bekannte Verbindung und kann beispielsweise gemäß dem Verfahren der EP-A-204 009 hergestellt werden.

Als Cyclisierungskatalysator ist kommerziell für die präparative Säulenchromatographie im Handel befindliches saures Aluminiumoxid, insbesondere Aluminiumoxid 90 aktiv, sauer, Aktivitätsstufe 1, für die Säulenchromatographie der Firma E. Merck, Darmstadt, das Aluminiumoxid mit der Bezeichnung ICN Alumina A, Aktivität 1 der Firma ICN Biomedicals, GmbH, oder äquivalente Produkte anderer Firmen.

Der Schmelzpunkt des Ausgangsdiols liegt bei 133°C, so daß beim Beginn der Reaktion die Temperatur erfindungsgemäß oberhalb dieser Temperatur liegen sollte, wodurch ein intensiver Kontakt mit dem Katalysator gewährleistet ist. Durch das sich bildende (-)3a,6,6,9a-Tetramethyl-perhydronaphtho[2,1-b]furan wird der Schmelzpunkt des Ausgangsdiols beträchtlich herabgesetzt. So hat das Ausgangsdiol z.B. in Gegenwart von etwa 20 % des Cyclisierungsproduktes nur noch einen Schmelzbereich von etwa 103 bis 107°C.

Daher arbeitet man mit Vorteil so, daß man die Umsetzung des Diols oberhalb seines Schmelzpunktes von 133°C startet und im Verlauf der Umsetzung die Temperatur langsam bis auf 80°C absinken läßt.

Prinzipiell kann man die Umsetzung aber auch bei Temperaturen unterhalb von 133°C starten, wenn man dem Reaktionsgemisch zu Beginn eine bestimmte Menge des Cyclisierungsproduktes zusetzt. Bei einem Zusatz von etwa 20 % des Cyclisierungsproduktes kann man das Ausgangsgemisch schon bei Temperaturen von etwa 107°C zum Schmelzen bringen und bei diesen milderen Temperaturen arbeiten. Nachteilig hierbei ist allerdings, daß sich bei diesen Temperaturen die benötigte Reaktionszeit verdoppelt.

Bevorzugt arbeitet man daher bei Temperaturen von 140°C bis etwa 120°C.

Überraschenderweise wird bei diesen Temperaturen in Gegenwart der beschriebenen Katalysatoren die gewünschte Produktqualität nicht beeinträchtigt, was bei Riechstoffen von entscheidender Bedeutung ist.

Die Reaktionszeiten des erfindungsgemäßen Verfahrens betragen bei diesen Temperaturen etwa 1 bis 6 Stunden, vorzugsweise 2 bis 4 Stunden.

Die Reaktionsgeschwindigkeit ist abhängig von der Reaktionstemperatur, so daß bei sehr hohen Temperaturen nur sehr kurze Verweilzeiten benötigt werden, und dadurch das erfindungsgemäße Verfahren auch kontinuierlich durchgeführt werden kann, ohne daß nennenswerte Einbußen an Selektivität bzw. an Geruchsqualität auftreten. Man arbeitet dann bei Temperaturen von 180 bis 200°C, vorzugsweise 185 bis 190°C und Reaktionszeiten von 5 bis 20 Minuten, vorzugsweise 10 bis 15 Minuten.

Nach Beendigung der Cyclisierungsreaktion wird das erhaltene Reaktionsgemisch entweder durch Destillation unter stark vermindertem Druck von dem Katalysator abgetrennt oder aber das Reaktionsgemisch in heißem Ethanol gelöst und der Katalysator durch Filtration abgetrennt.

Das Verfahrensprodukt der Formel Ia wird aus der ethanolischen Lösung in an sich bekannter Weise durch Kristallisation isoliert. Die Isolierung aus ethanolischer Lösung ist sehr vorteilhaft, da Ethanol in der Parfümindustrie ohnehin als Standardlösungsmittel Verwendung findet. Die erzielten Ausbeuten sind im allgemeinen größer als 90 % der Theorie bei Reinheiten von über 99 %.

Ein weiterer Vorteil des erfindungsgemäßen Verfahrens ist, daß der Katalysator durch Auswaschen mit Ethanol regeneriert und danach wieder verwendet werden kann. Mit Vorteil wird der nach Abtrennen der ethanolischen Lösung des Reaktionsgemisches anfallende, noch etwa 70°C warme Katalysator 1 bis 3 mal mit heißem Ethanol gewaschen und kann dann erneut als Katalysator verwendet werden (vgl. Beispiel 3). Bei der erneuten Verwendung kann der Katalysator gleich ethanolfeucht eingesetzt werden, da beim Anheizen des Reaktionsgemisches unter vermindertem Druck das Ethanol noch vor Einsetzen der Wasserabspaltung abdestilliert.

Mit Hilfe des erfindungsgemäßen Verfahrens ist es möglich, Decahydro-2-hydroxy-2,5,5,8-tetramethyl-1-naphthalinethanol auf sehr einfache und trotzdem kostengünstige und umweltschonende Weise in sehr guten Ausbeuten stereoselektiv in das als Duftstoff sehr begehrte (-)3a,6,6,9a-Tetramethyl-perhydronaphtho[2,1-b]furan zu überführen.

### Beispiel 1

In einen Vierhals-Rührkolben (250 ml), der mit einer Destillationsbrücke versehen war, wurden 50,8 g (0,2 mol) Ambroxdiol und 25,0 g Aluminiumoxid 90 aktiv, sauer, Aktivitätsstufe 1 (Firma Merck) eingefüllt und das Reaktionsgemisch nach Evakuieren mit einer Wasserstrahlpumpe auf etwa 30 - 40 mm Hg unter Rühren langsam mittels eines Paraffinölbads auf Temperaturen von etwa 140 bis 145°C (Kolbeninnentemperatur) erhitzt. Bei einer Temperatur von 133°C begann das Produkt zu schmelzen. Das bei der Reaktion gebildete Wasser wurde über die Destillationsbrücke abdestilliert. Nach 90 Minuten (min) war die Umsetzung beendet. Anschließend wurde die Ölbadheizung ausgeschaltet, das Gemisch auf 75 bis 80°C abgekühlt und auf Normaldruck gebracht. Danach wurden 90 ml Ethanol zugefügt, das Reaktionsgemisch 30 min unter Rückfluß zum Sieden erhitzt, dann auf etwa 50 bis 55°C abgekühlt und der Katalysator mittels einer G4-Glasfritte abgetrennt und mit 60 ml Ethanol gewaschen. Das Filtrat wurde mit dem Waschethanol vereinigt, die Lösung unter Rückfluß zum Sieden erhitzt und dann Wasser zugetropft, bis eine deutliche Trübung bei Siedetemperatur eintrat, wozu etwa 75-80 ml Wasser notwendig waren. Unter Rühren wurde langsam abgekühlt und letztlich bei 10°C mittels einer G3-Glasfritte das auskristallisierte Produkt isoliert, mit 100 ml eines Ethanol-Wasser-Gemisches (2:1) gewaschen und im Trockenschrank bei ca. 20 mbar und 55-60°C bis zur Gewichtskonstanz getrocknet. Die Ausbeute betrug 43 g (entsprechend 91,4 % der Theorie (d.Th.)) mit einem Festpunkt von 75-77°C. Gemäß GC-Analyse war die Reinheit des Produktes größer als 99%.

### Beispiel 2

203,2 g (0,8 mol) Ambroxdiol und 100,0 g Aluminiumoxid ICN Alumina A, Aktivität 1 (Firma ICN Biomedicals GmbH) wurden in einen 1-Liter-Kolben gefüllt, der mit 4 schräg verlaufenden Schikanen versehen ist. Der Kolben mit dem Reaktionsgemisch wurde am Rotavapor in ein auf 140°C vorgeheiztes Ölbad getaucht und bei 24 U/min und 20-24 mm Hg das entstandene Reaktionswasser abdestilliert. Nach einer Reaktionszeit von 4,5 Stunden (h) bei 140°C und 20 mbar war die Umsetzung vollständig. Nach Abkühlen auf ca. 85°C wurde der Katalysator mittels eines auf 80°C vorgewärmten Druckfilters abgetrennt und mit ca. 300 ml Ethanol gewaschen. Filtrat und Waschethanol wurden vereinigt und unter Rückfluß zum Sieden erhitzt. Zu der klaren heißen Lösung wurde Wasser zugesetzt bis eine deutliche Trübung auftrat, wozu etwa 140 ml Wasser notwendig waren. Durch langsames Abkühlen unter Rühren kristallisiert das Wertprodukt in balkenähnlichen Kristallen aus. Die Kristallmaische wurde bei 10°C über Nacht im Kühlschrank aufbewahrt und anschließend mittels einer G3-Glasfritte isoliert und mit einem Ethanol/Wasser-Gemisch (1:1) gewaschen und trocken gesaugt. Durch Trocknen bei 55°C unter Wasserstrahlpumpen-Vakuum bis zur Gewichtskonstanz erhielt man 175 g (entsprechend 92,5% d.Th.) eines Produktes, welches nach GC-Analyse eine Reinheit von 100 % aufwies.

Durch Waschen des noch heißen Katalysators mit Ethanol erlangt dieser seine alte Aktivität zurück, so daß er mehrfach erfindungsgemäß eingesetzt werden kann. Der Katalysator kann dann gleich ethanolfeucht eingesetzt werden, da beim Anheizen des Reaktionsgemisches unter vermindertem Druck das Ethanol abdestilliert bevor die Wasserabspaltung einsetzt.

### Beispiel 3

50,8 g (0,2 mol) Ambroxdiol und 10 g Aluminiumoxid 90, sauer (Aktivitätsstufe 1 der Firma Merck) wurden in einem 250 ml Rundkolben, der mit einer Destillationsbrücke versehen war, zusammengegeben. Unter Rühren wurde das Gemisch langsam bei 2,5 bis 3 mbar auf 135 bis 140°C und das sich bildende Reaktionswasser abdestilliert. Nach ca. 30 min wurde die Temperatur auf 160°C gesteigert und bei dem Druck von 2,5 bis 3 mbar das (-)3a,6,6,9-Tetramethyl-perhydronaphtho[2,1-b]furan abdestilliert.

Die Ausbeute betrug 45,2 g entsprechend 95,8 % d.Th. Das Produkt wies nach GC-Analyse eine Reinheit von 94,14 auf. Der Schmelzpunkt betrug 68-72°C.

### Beispiel 4

203,2 g Ambroxdiol wurden analog Beispiel 2 mit 100 g neuem ICN Alumina A, Aktivität 1, sowie mit 1 bis 3 mal durch Waschen des noch heißen Katalysators mit Ethanol regeneriertem ICN Alumina A, Aktivität 1, unter den in der folgenden Tabelle angegebenen Reaktionsbedingungen zu (-)3a,6,6,9a-Tetramethyl-perhydronaphtho[2,1-b]furan umgesetzt. In der Tabelle sind die jeweils erzielten Ausbeuten und Reinheiten des Produktes angegeben.

**Tabelle**

| Beispiel | Katalysator | Reaktionstemperatur [°C] | Reaktionszeit [h] | Druck [mbar] | Reiheit (GC) [%] | Ausbeute | |
|---|---|---|---|---|---|---|---|
| | | | | | | [g] | [% d. Th.] |
| 4a | ICN Alumina A, aktiv 1, neu | 140 | 3 | 25-20 | 99,9 | 172,0 | 90,95 |
| 4b | 1x regeneriert | 138-140 | 4,5 | 20 | 100 | 172,0 | 90,95 |
| 4c | 2x regeneriert | 138-140 | 5,0 | 20 | 100 | 172,0 | 90,95 |
| 4d | 3x regeneriert | 140 | 5,5 | 22 | 99,95 | 172,0 | 90,95 |

## Patentansprüche

1. Verfahren zur stereoselektiven Herstellung von (-)3a,6,6,9a-Tetramethyl-perhydronaphtho[2,1-b]furan der Formel Ia 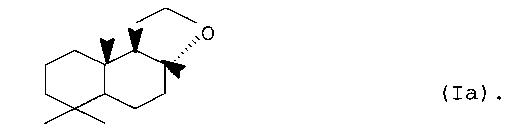 durch Dehydratisierung und Cyclisierung von Decahydro-2-hydroxy-2,5,5,8-tetramethyl-1-naphthalinethanol mittels festen sauren Katalysatoren, dadurch gekennzeichnet, daß man das Decahydro-2-hydroxy-2,5,5,8-tetramethyl-1-naphthalinethanol in geschmolzenem Zustand in Gegenwart von 10 bis 100 Gew-%, bezogen auf das Diol, an einem kommerziell für die (präparative) Säulenchromatographie angebotenen aktiven sauren Aluminiumoxid auf Temperaturen zwischen 80 bis 200°C erhitzt.

2. Verfahren gemäß Anspruch 1, dadurch gekennzeichnet, daß man die Umsetzung des geschmolzenen Decahydro-2-hydroxy-2,5,5,8-tetramethyl-1-naphthalinethanols in Gegenwart von Aluminiumoxid 90 aktiv sauer (Aktivitätsstufe I) für die Säulenchromatographie der Firma E. Merck, Darmstadt, durchführt.

3. Verfahren gemäß Anspruch 1, dadurch gekennzeichnet, daß man das geschmolzene Decahydro-2-hydroxy-2,5,5,8-tetramethyl-1-naphthalinethanol in Gegenwart von Aluminiumoxid der Bezeichnung ICN Alumina A, Akt. 1, der Firma ICN Biomedicals GmbH erhitzt.

4. Verfahren gemäß Anspruch 1, dadurch gekennzeichnet, daß man die Umsetzung des Decahydro-2-hydroxy-2,5,5,8-tetramethyl-1-naphthalinethanols oberhalb seines Schmelzpunktes von 133°C startet und im Verlauf der Umsetzung die Temperatur langsam bis auf 80°C absinken läßt.

5. Verfahren gemäß Anspruch 1, dadurch gekennzeichnet, daß man das geschmolzene Decahydro-2-hydroxy-2,5,5,8-tetramethyl-1-naphthalinethanol für 1 bis 6 Stunden auf Temperaturen von 80 bis 140°C erhitzt.

6. Verfahren gemäß Anspruch 1, dadurch gekennzeichnet, daß man das geschmolzene Decahydro-2-hydroxy-2,5,5,8-tetramethyl-1-naphthalinethanol für 5 bis 20 Minuten auf Temperaturen von 180 bis 200°C erhitzt.

7. Verfahren gemäß Anspruch 1, dadurch gekennzeichnet, daß man das geschmolzene Decahydro-2-hydroxy-2,5,5,8-tetramethyl-1-naphthalinethanol in Gegenwart von einfach oder mehrfach durch Waschen mit Ethanol regeneriertem Aluminiumoxid umsetzt.

8. Verfahren gemäß Anspruch 1, dadurch gekennzeichnet, daß man das bei der Umsetzung erhaltene Reaktionsgemisch in heißem Ethanol löst, den Katalysator abtrennt und das Reaktionsprodukt aus der ethanolischen Lösung isoliert.

9. Verfahren gemäß Anspruch 1, dadurch gekennzeichnet, daß man das bei der Umsetzung erhaltene Reaktionsgemisch durch Destillation unter stark vermindertem Druck vom Katalysator abtrennt.
